# EUROPEAN PATENT APPLICATION

(11) **EP 4 556 569 A1**
(43) Date of publication of application: **21.05.2025**
(21) Application number: 22954117.2
(22) Date of filing: 09.11.2022
(51) Int. Cl.: C12P 19/02, C12P 19/04, C12P 19/14, C12N 9/38, C12R 1/01

(54) **METHOD FOR LIQUEFYING AGAROSE AND PRODUCING NEOAGAROTETRAOSE/NEOAGAROHEXAOSE BY USING THERMOSTABLE GH16B BETA-AGARASE DERIVED FROM NOVEL AGAR-DEGRADING BACTERIUM**

(30) Priority: 02.08.2022 KR 20220095807
(71) Applicant: Kyungpook National University Industry-Academic Cooperation Foundation, Daegu 41566 (KR)
(72) Inventor: KIM, Young Ho, Gunwi-gun, Gyeongsangbuk-do 39034 (KR); LEE, Hee Kyoung, Eumseong-gun, Chungcheongbuk-do 27675 (KR); JANG, Won Young, Daegu 41267 (KR)
(74) Representative: Meissner Bolte Partnerschaft mbB
(86) International application number: PCT/KR2022/017585
(87) International publication number: WO 2024/029662

(57) **Abstract**

The present invention relates to a method of producing neoagarotetraose (NA4) and neoagarohexaose (NA6) with high purity in large amounts by liquefaction of the substrate agarose using a thermostable GH16B β-agarase derived from a novel agarolytic bacterium *Cell vibrio* sp. KY-GH-1 deposited under accession number KCTC 13629BP. The method of producing neoagarotetraose (NA4) and neoagarohexaose (NA6) with high purity in large amounts by liquefaction of the substrate agarose using a thermostable GH16B β-agarase derived from a novel agarolytic bacterium *Cell vibrio* sp. KY-GH-1 deposited under accession number KCTC 13629BP according to the present invention may effectively produce NA4 and NA6 with high purity in large amounts by efficiently performing liquefaction of the substrate agarose, because the thermostable GH16B β-agarase exhibits excellent enzyme activity at a high temperature equal to or higher than the agarose gelling temperature and has long-term thermal stability even at high temperatures.

## Description

### Technical Field

The present invention relates to a method of producing neoagarotetraose (NA4) and neoagarohexaose (NA6) with high purity in large amounts by liquefaction of the substrate agarose using a thermostable GH16B β-agarase derived from a novel agarolytic bacterium *Cell vibrio* sp. KY-GH-1 deposited under accession number KCTC 13629BP.

### Background Art

Agar is a complex polysaccharide present in the cellular walls of marine red algae such as *Gelidium, Gracilariais,* and *Gelidiella,* and mainly consists of about 60 to 70% agarose and 30 to 40% agaropectin. Agarose is a polymer composed of alternating α-1,3-linked 3,6-anhydro-L-galactose (L-AHG) and β-1,4-linked D-galactose, and agaropectin consists of the same agarobiose repeating units, but some of the sugar residues are replaced by sulfuric acid ester, pyruvic acid, and ethers. Agar has excellent gelling ability to form a stable gel matrix that is resistant to microbial degradation, and thus is widely used in microbial culture media and food industries.

Several genera of marine bacteria and few non-marine bacteria are known to degrade agar into monosaccharides that may be used as the sole carbon source. To assimilate agar as a sole carbon source, an agarolytic bacterium produces a combination of different agarases for efficient and complete agar hydrolysis into L-AHG and D-galactose. Two representative types of agarase, that is, β-agarase (EC 3.2.1.81) that cleaves β-1,4-glycosidic linkages, and α-agarase (EC 3.2.1.158) that cleaves α-1,3-glycosidic linkages, have been reported. Most of the agarases reported to date belong to the endo-β-agarases that produce neoagaro-oligosaccharides (NAOS) from agarose degradation. There are few reports on α-agarases that produce agaro-oligosaccharides (AOSs) from agarose. Bacterial agarases are classified into different glycoside hydrolase (GH) families based on similarities in their amino acid sequences. These include GH16, GH50, GH86, and GH118, which act as β-agarases, as well as GH96 and GH117, which act as α-agarases [Flatment et al., 2007; Ha et al., 2011; Lee et al., 2019; Jang, 2021]. Each agarase degrades agarose in a distinct manner. That is, GH16, GH86 and GH118 agarases endolytically degrade agarose into neoagarotetraose (NA4)/neoagarohexaose (NA6), NA6/neoagarooctaose (NA8), and NA8/neoagarodecaose (NA10), respectively. The β-agarases of GH50 family degrade agarose via exolytic activity or a combination of exolytic and endolytic activities to produce neoagarobiose (NA2), NA4, or NA2/NA4 as end products. GH96 α-agarases hydrolyze agarose primarily into agarotetraose (A4), whereas GH117 α-neoagarobiose hydrolases (α-NABH) hydrolyzes NA2 into L-AHG and D-galactose.

A number of studies have reported various biological activities of agar degradation products (NAOS, AOS, NA2, and L-AHG) produced by microbial enzymes or acid hydrolysis. These activities include antioxidant activity, prebiotic activity, antitumor activity, anti-inflammatory activity, anti-diabetes and anti-obesity activity, anticariogenic activity, and skin moisturizing and whitening activities. Furthermore, it has been reported that oral administration of NA4 in a mouse model protects against fatigue and liver damage, induced by intense exercise, by modulating the gut microbiota.

In this context, various studies have been conducted to characterize agarases, which are obtained as purified or recombinant proteins from agarolytic bacteria, and to develop individual enzymatic processes for producing L-AHG, NA2, NA4, NA6, and NAOSs from agarose. However, the saccharification processes that enzymatically degrade agarose into the monosaccharides L-AHG and D-Gal need to be further improved. To this end, there is an urgent need for further research on thermostable GH16 family β-agarases that can efficiently hydrolyze agarose, liquefied by heat treatment, to yield NAOSs with a low degree of polymerization (DP). This is because when the thermostable endolytic GH16 family β-agarases that liquefy agarose to produce low-DP NAOSs are not only prerequisites for a more desirable agarose saccharification process that can efficiently produce NA2 when combined with exolytic GH50 β-agarase, but also prerequisites for a saccharification process that can ultimately produce L-AHG/D-Gal when combined with GH50 β-agarase and α-NABH.

### DISCLOSURE

### Technical Problem

The present invention has been made in order to solve the above-described problems occurring in the prior art, and the problem to be solved by the present invention is to provide a method of producing NA4/NA6 with high purity in large amounts by liquefaction of the substrate agarose using a highly thermostable GH16B β-agarase derived from a *Cellvibrio* sp. KY-GH-1 strain deposited under accession number KCTC 13629BP.

### Technical Solution

To solve the above problem, the present invention provides a method for producing neoagarotetraose (NA4) and neoagarohexaose (NA6), characterized by enzymatically degrading a substrate by treating the same with a GH16B β-agarase comprising SEQ ID NO: 1.

The GH16B β-agarase comprising SEQ ID NO: 1 is preferably derived from a *Cellvibrio* sp. KY-GH-1 strain deposited under accession number KCTC 13629BP.

The substrate is preferably agarose or neoagarooligosaccharides (NAOS).

The concentration of the agarose is preferably 9 wt% or less.

The treatment is preferably performed at a temperature ranging from 40 to 60°C.

The treatment is preferably performed at a pH ranging from pH 5.0 to 8.0.

The treatment is preferably performed by further adding Mn²⁺.

The Mn²⁺ is preferably MnCl₂ or MnSO₄.

The concentration of the MnCl₂ or MnSO₄ is preferably 0.5 to 2.5 mM.

The treatment is preferably performed by further adding tris(2-carboxyethyl)phosphine (TCEP).

The concentration of the tris(2-carboxyethyl)phosphine is preferably 5 to 15 mM.

### Advantageous Effects

The method of producing neoagarotetraose (NA4) and neoagarohexaose (NA6) with high purity in large amounts by liquefaction of the substrate agarose using a thermostable GH16B β-agarase derived from a novel agarolytic bacterium *Cellvibrio* sp. KY-GH-1 deposited under accession number KCTC 13629BP according to the present invention may effectively produce NA4 and NA6 with high purity in large amounts by efficiently performing liquefaction of the substrate agarose, because the thermostable GH16B β-agarase exhibits excellent enzyme activity at a high temperature equal to or higher than the agarose gelling temperature and has long-term thermal stability even at high temperatures.

### Brief Description of Drawings

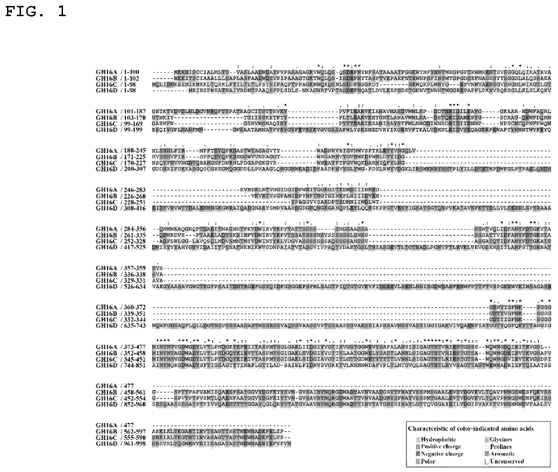

FIG. 1 shows a comparison of the amino acid sequences of GH16A, GH16B, GH16C, and GH16D β-agarases from *Cellvibrio* sp. KY-GH-1. Amino acids are displayed as one-letter abbreviations after aligning the open reading frames of the amino acid sequences for maximal identity using Clustal X. Identical residues among the two sequences are indicated by asterisks above the column. Amino acid residues are highlighted using Clustal X color scheme. Deletions are indicated by dashes

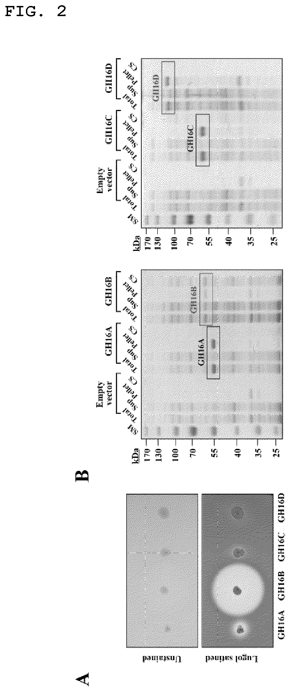

FIG. 2 shows the results of detecting recombinant His-tagged GH16A, GH16B, GH16C, and GH16D β-agarases, expressed in *E. coli,* by Lugol's iodine solution and SDS-PAGE. (A) Transformants expressing recombinant His-tagged GH16A, GH16B or GH16C β-agarases were spotted onto LB agarose plates (containing 1.5% agarose, 50 µg/mL chloramphenicol, 25 µg/mL kanamycin and 0.1 mM IPTG) and incubated for 3 days at 37°C. Plates were stained with Lugol's iodine solution at room temperature. (B) Total (T), soluble (S) supernatant, and insoluble inclusion (P) intracellular fractions as well as culture supernatants (CS) as extracellular fractions were prepared from cultured *E. coli* BL21(DE3) pLysS transformants cultured in the presence of 0.5 mM IPTG. Equivalent amounts of each portion were electrophoresed SDS-polyacrylamide gel. Representative results are shown, and two additional experiments yielded similar results.

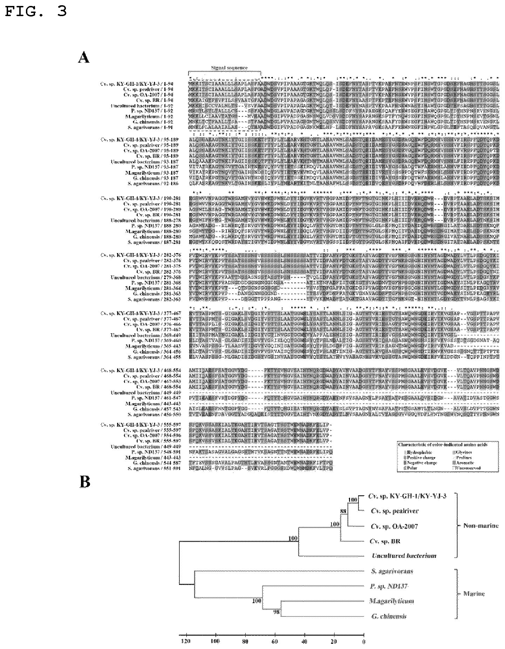

FIG. 3 shows multiple amino acid sequence alignment comparing GH16B β-agarase with nine GH16 family β-agarases available in the Genbank database (A) and their phylogenetic relationship (B). Amino acids are designated using one-letter abbreviations after the alignment of the open reading frames of amino acid sequences for maximal identity using Clustal X. Identical residues in all sequences are indicated by asterisks above the column. Conserved substitutions are indicated by colons, semiconserved substitutions are indicated by dots, and conserved residues are highlighted using Clustal X color scheme. Deletions are indicated by dashes. The unrooted phylogenetic tree was constructed using UPGMA method. Numbers at nodes represent the levels of bootstrap support.

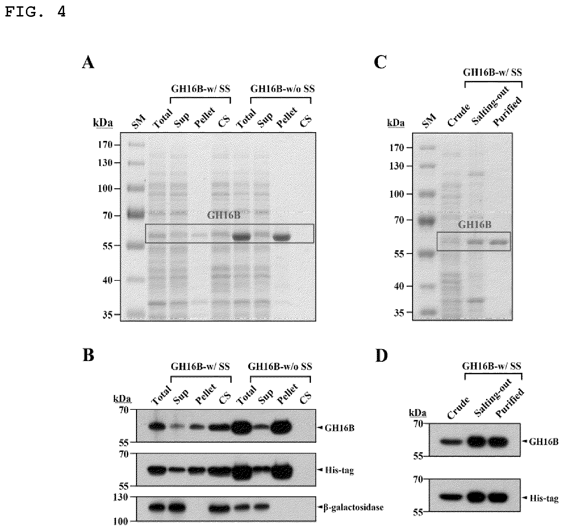

FIG. 4 shows the results of SDS-PAGE and Western blot analysis of recombinant His-tagged GH16B β-agarase with or without N-terminal 22-aa signal sequence in individual intracellular fractions and culture supernatants of E. *coli* transformants (A), and GH16B β-agarase purified from the culture supernatants (B). Lane: SM, prestained size markers; GH16B-w/SS, GH16B β-agarase with the signal sequence; GH16B-w/oSS, GH16B β-agarase without the signal sequence; crude GH16B-w/SS, enzyme in the culture supernatants; salting-out GH16B-w/SS, enzyme concentrated using ammonium sulfate salting-out of the culture supernatants; purified GH16B-w/SS, enzyme purified from the concentrated GH16B-w/SS using the Ni-NTA purification system.

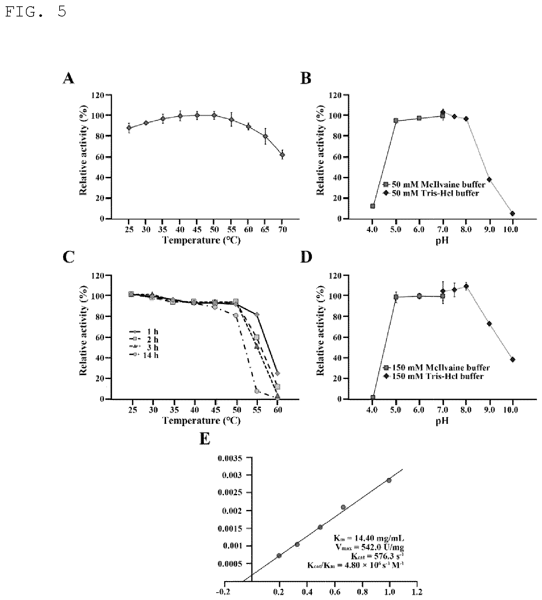

FIG. 5 shows the biochemical characteristics of GH16B β-agarase. (A and b) The effects of the optimal temperature and pH on the enzymatic activity were determined using 0.4% agarose and 0.8 µg/mL of the purified enzyme. Each value is expressed as the mean±SEM (n=3; three replicates per independent experiment). (C and D) The temperature and pH stabilities of the purified GH16B β-agarase were measured following incubation for the indicated time at various temperatures and incubation for 4 hours at various pH values. Data are expressed as the mean±SEM (n=3; three replicates per independent experiment). (E) Lineweaver-Burk plot was used to determine the *Km* and *Vmax* values of the purified GH16B β-agarase based on indicated concentrations of the enzyme and the substrate agarose. Representative results are shown, and two additional experiments yielded similar results.

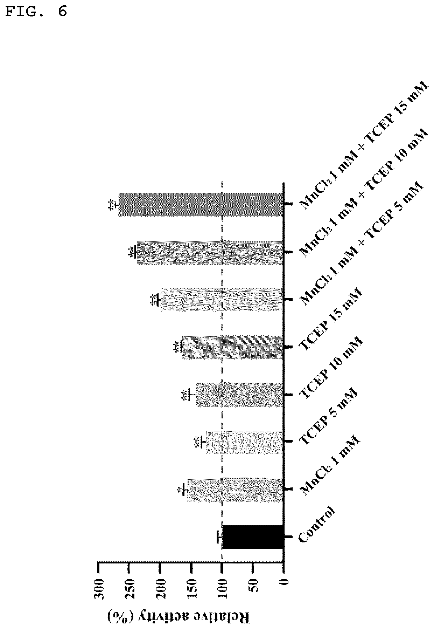

FIG. 6 shows the effects of MnCl₂ and TCEP on the GH16B β-agarase activity. Individual and co-treatment effects of MnCl₂ (1 mM) and TCEP (5, 10, or 15 mM) on the activity of GH16B β-agarase were investigated by incubating the purified enzyme (0.8 µg/mL) with 0.4% agarose in 50 mM Tris-HCl buffer (pH 7.0) at 50°C for 30 min. The enzymatic activity measured in the absence of MnCl₂ and TCEP was defined as 100%.

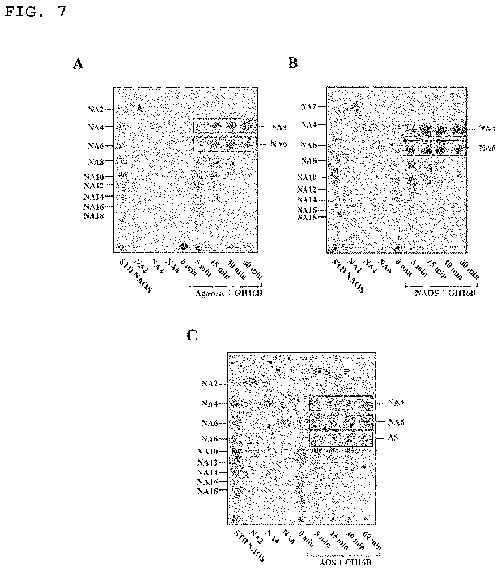

FIG. 7 shows the results of time-kinetic analysis of GH16B β-agarase-catalyzed hydrolysates of agarose, NAOS, and AOS using TLC. For hydrolysis of each substrate, 0.4% agarose (A), 1.0% NAOS (B), or 1.0% AOS (C) was treated with the enzyme (0.8 µg/mL) in 50 mM Tris-HCl buffer (pH 7.0) at 50°C for the indicated time. Standard neoagarooligosaccharides (STD NAOS) and agaro-oligosaccharides (AOSs) were prepared as described in the following Examples. Equivalent amounts of each sample were spotted on TLC plates and developed with *n*-butanol-ethanol-H₂O [3:2:2(v/v)). Oligosaccharides were visualized by spraying an ethanol solution containing 0.2% (w/v) naphthoresorcinol and 10% (v/v) H₂SO₄, followed by heating at 80°C. Representative results are shown, and two additional experiments yielded similar results.

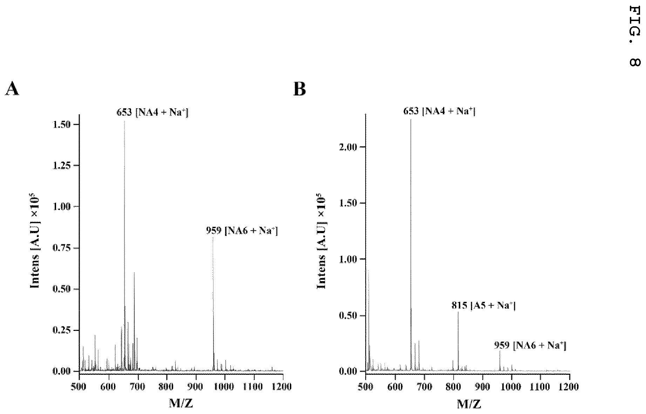

FIG. 8 shows the results of analysis of GH16B β-agarase-catalyzed hydrolysates of agarose and AOS using MALDI-TOF/TOF MS. For agarose hydrolysis, 0.4% agarose was treated with 0.8 µg/mL GH16B β-agarase in 50 mM Mcllvaine buffer (pH 7.0) at 50°C for 60 min. For AOS hydrolysis, 1.0% AOS was treated with 0.8 µg/mL GH16B β-agarase in 50 mM Mcllvaine buffer (pH 7.0) at 50°C for 60 min. MALDI-TOF/TOF MS analysis was performed as described in the following Examples.

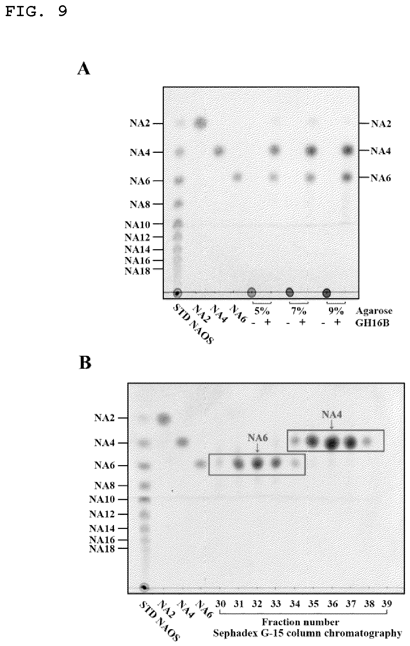

FIG. 9 shows the results of GH16B β-agarase-catalyzed complete hydrolysis of agarose to NA4 and NA6, and purification of NA4 and NA6 from the hydrolysate via Sephadex G-15 column chromatography. (A) After agarose at various concentrations (5.0%, 7.0%, or 9.0% in 50 mM Mcllvaine buffer (pH 7.0) was treated with GH16B β-agarase (1.6 µg/mL) under optimum reaction conditions (5 mM MnCl₂, 10 mM TCEP, 50 mM Mcllvaine buffer, pH 7.0) and continuous magnetic stirring at 50°C for 14 hours, and then the hydrolysates were analyzed by TLC to identify the conversion of agarose to NA4 and NA6. (B) For purification of NA4 and NA6 from the hydrolysate obtained by the catalytic action of GH16B β-agarase, 9.0% agarose (20 mL) was treated with the enzyme (1.6 µg/mL) under optimal conditions for 14 hours and then freeze-dried. The freeze-dried sample was subjected to Sephadex G-15 column chromatography using DI water as a mobile phase. Equivalent volumes of individual fractions were analyzed by TLC as described in the following Examples below. Representative results are shown, and two additional experiments yielded similar results.

### Best Mode

Hereinafter, the present invention will be described in detail.

By sequencing the genome of a freshwater agarolytic bacterium *Cellvibrio* sp. KY-GH-1 (KCTC 13629BP), the inventors of the present invention recently reported the genes encoding agarases that hydrolyze agar into L-AHG and D-galactose. The KY-GH-1 strain was shown to possess genetic information on four endolytic β-agarases [GH16A (SEQ ID NO: 2), GH16B (SEQ ID NO: 1), GH16C ((SEQ ID NO: 3), and GH16D (SEQ ID NO: 4)] in a 77-kb agarase gene cluster. However, it remains unknown which of the four isoenzymes possesses the most prominent endolytic GH16 β-agarase activity in agarose liquefaction and production of NAOS such as NA4/NA6 by agarose liquefaction.

In the present invention, four recombinant His-tagged GH16 family β-agarases (GH16A, GH16B, GH16C, and GH16D) derived from *Cellvibrio* sp. KY-GH-1 were produced using a pET-30a vector and an *E. coli* expression system, and their enzymatic activities were compared. As a result, it was found that, among these four recombinant GH16 family β-agarases, GH16B β-agarase had the highest endolytic β-agarases activity. In addition, the enzymatic properties of recombinant GH16B β-agarase were investigated, and the efficiency of NA4/NA6 production through agarose liquefaction using the enzyme was investigated.

Specifically, the inventors of the present invention produced by expression of four GH16 family β-agarases (GH16A, GH16B, GH16C, and GH16D), derived from the freshwater agarolytic bacterium *Cellvibrio* sp. KY-GH-1, using an *E. coli* expression system, and compared their enzymatic activities. Among these four GH16 family β-agarases, only GH16B (597 amino acids, 63.8 kDa) having an N-terminal 22-amino acid signal sequence was secreted into the extracellular medium and efficiently endolytically hydrolyzed agarose produce neoagarotetraose (NA4) and neoagarohexaose (NA6) as end products. The optimal temperature and pH for the recombinant GH16B β-agarase were shown to be 50°C and 7.0, respectively. Also, this enzyme was stable up to 50°C and over a pH range of 5.0 to 8.0. The *Km, Vmax, kcat,* and *kcat*/*Km* values of the enzyme for agarose were 14.40 mg/mL, 542.0 U/mg, 576.3 s⁻¹, and 4.80×10⁶ s⁻¹ M⁻¹, respectively. The co-addition of 1 mM MnCl₂ and 15 mM tris(2-carboxyethyl)phosphine (TCEP) was shown to enhance the enzymatic activity by 2.6 times. Enzyme-catalyzed hydrolysis of agarose or neoagaro-oligosaccharides (NAOS) mainly produced NA4/NA6, whereas enzyme-catalyzed hydrolysis of agaro-oligosaccharides (AOSs) also produced agaropentaose (A5) along with NA4/NA6. When agarose melted by heat treatment was treated with recombinant GH16B β-agarase (1.6 µg/mL) under continuous magnetic stirring at 50°C for 14 hours, the agarose could be efficiently liquefied up to a concentration of 9%. Purification of NA4/NA6 from the agarose hydrolysate (9% agarose, 20 mL) via Sephadex G-15 column chromatography yielded about 650 mg NA4/about 900 mg NA6 (i.e., about 85.3% of the theoretical maximum yield). These results suggest that the recombinant thermostable GH16B β-agarase is useful for agarose liquefaction to produce NA4/NA6.

Therefore, the present invention provides a method for producing neoagarotetraose (NA4) and neoagarohexaose (NA6), characterized by enzymatically degrading a substrate by treating the same with a GH16B β-agarase comprising SEQ ID NO: 1.

The GH16B β-agarase comprising SEQ ID NO: 1 is preferably derived from a *Cellvibrio* sp. KY-GH-1 strain deposited under accession number KCTC 13629BP.

The GH16B β-agarase is preferably obtained from an *E*. *coli* transformant into which a GH16B β-agarase gene has been introduced.

The GH16B β-agarase gene is preferably introduced into *E. coli* after cloned into an expression vector and introduced.

The substrate is preferably agarose or neoagarooligosaccharides (NAOS).

The concentration of the agarose is preferably 9 wt% or less. In a preferred embodiment, the concentration of the agarose may be, for example, 1 to 9 wt% or 5 to 9 wt%.

The treatment is preferably performed at a temperature ranging from 40 to 60°C. In a preferred embodiment, the temperature may be 50°C.

The treatment is preferably performed at a pH ranging from pH 5.0 to 8.0. In a preferred embodiment, the pH may be 7.0.

The treatment is preferably performed by further adding Mn²⁺.

The Mn²⁺ is preferably MnCl₂ or MnSO₄.

The concentration of the MnCl₂ or MnSO₄ is preferably 0.5 to 2.5 mM. In a preferred embodiment, the concentration of the MnCl₂ or MnSO₄ may be 1 mM.

The treatment is preferably performed by further adding tris(2-carboxyethyl)phosphine (TCEP).

The concentration of the tris(2-carboxyethyl)phosphine is preferably 5 to 15 mM. In a preferred embodiment, the concentration of the TCEP may be 5 mM, 10 mM or 15 mM.

### Mode for Invention

Hereinafter, the present invention will be described in more detail by way of specific examples. The following examples are intended to describe preferred specific examples of the present invention, and it is obvious that the scope of the present invention should not be construed as being limited by the matters described in the following examples.

### [Examples]

### 1. Materials and Methods

### 1.1. Materials

Naphthoresorcinol, D-galactose, isopropyl β-D-1-thiogalactopyranoside (IPTG), tris(2-carboxyethyl)phosphine (TCEP), 3,5-dinitrosalicylic acid (DNS), Coomassie brilliant blue (CBB) R-250, ethylenediamine tetraacetic acid (EDTA), chloramphenicol, kanamycin, complete Freund's adjuvant, and sodium dodecyl sulfate (SDS) were purchased from Sigma-Aldrich (St. Louis, MO, USA). Restriction enzymes (NedI and *Xho*I) and T4 ligase were purchased from Roche (Basel, Switzerland). The pET-30a vector for expressing C-terminal 6x His-tagged protein and Immobilon-P membrane were purchased from EMD Millipore Corporation (Temecula, CA, USA). *E. coli* BL21 (DE3) was purchased from Novagen (Madison, WI, USA). Micro BCA kits were purchased from Pierce (Rockford, IL, USA), and silica Gel 60 aluminum thin layer chromatography (TLC) plates coated with fluorescent indicator F254 were purchased from Merck (Darmstadt, Germany). PageRuler Prestained Protein Ladder and Ni-NTA resin were purchased from ThermoFisher Scientific (Rockford, IL, USA). A NAOS standard mixture containing NA2-NA18 was prepared as described previously and provided by Dr. Sang-Hyeon Lee. An AOS mixture was prepared by mild acid hydrolysis of 1% agarose in 3 M acetic acid treated at 80°C for 3 hours, then adjusting the pH to 7.0 using 5 M NaOH. After freeze-drying, the solid sample was washed three times with 95% (v/v) cold ethanol and then dried using a rotary evaporator to obtain AOS powder.

### 1.2. Cloning and Expression of GH16 β-Agarase Genes using E. coli Expression System

Four GH16 β-agarase genes (GH16A, GH16B, GH16C, and GH16D) in in the genomic DNA of the *Cellvibrio* sp. KY-GH-1 strain were amplified by PCR using the following primers: NdeI-forward primers [5'-GCGGCATA-TGAAAAAAATCACTTCATGTA-3' (SEQ ID NO: 5) for GH16B; 5'-GCGGCATATGCAGTTAATAGATAATAAAGAG-3' (SEQ ID NO: 6) for GH16C; 5'-GCGGCATATGCGTATTCGCTCACCTTG-3' (SEQ ID NO: 7) for GH16D; 5'-CGCATATGGCCGATTGGGATTCAGTTCC-3' (SEQ ID NO: 8) for GH16B without a signal peptide sequence]; BamHI-forward primer [5'-CGGGATCCATGAAAAAGCATATTTCATGCTG-3' (SEQ ID NO: 9) for GH16A]; XhoI-reverse primers [5'-CTAACTCGAGGGGTATCAATTCAAACTTG-3' (SEQ ID NO: 10) for GH16B without a signal peptide sequence; 5'-CGCGCTCGAGGGGTACTAATTCAAATTTATCC-3' (SEQ ID NO: 11) for GH16C; 5'-CGCGCTCGAGATTTACTGGCACAAACTCAATG-3' (SEQ ID NO: 12) for GH16D]; and HindIII-reverse primer [5'-CGAAGCTTGAGTGAGCCAACTCGAGTAA-3' (SEQ ID NO: 13) for GH16A]. The PCR products were digested with appropriate restriction enzyme sets and ligated into the pET-30a vector for expression of the C-terminal 6x his-tagged protein using T4 ligase. The recombinant pET-30a plasmids were transformed into *E. coli* BL21 (DE3) pLysS. The transformants containing the GH16 β-agarase gene were cultured overnight at 30°C on LB plates containing 50 µg/mL chloramphenicol and 25 µg/mL kanamycin.

Expression of each β-agarase gene inserted into the pET-30a plasmid in *E. coli* BL21 (DE3) pLysS was induced as described previously. Briefly, each transformant was cultured at 25°C in LB medium containing 50 µg/mL chloramphenicol and 25 ug/mL kanamycin with shaking, and when OD₆₀₀ reached 0.4 to 0.6, 0.5 mM IPTG was added to induce production of recombinant GH16 β-agarase protein. The cells were cultured overnight at 20°C. To identify the recombinant enzyme proteins produced by the transformants and to determine whether they were secreted extracellularly, the cell culture was fractionated into four fractions (total, soluble, and insoluble cell fractions) as described previously, and the extracellular culture supernatant was also recovered. Equal amounts of each fraction were electrophoresed on an 8% SDS-polyacrylamide gel. The recombinant enzymes were visualized by staining with CBB.

### 1.3. Zymogram for Agarase Activity on LB Agarose Plates

*E. coli* BL21 (DE3) pLysS transformants harboring the recombinant pET-30a plasmid containing GH16A, GH16B, GH16C, or GH16D were spotted onto LB agarose plates (containing 1.5% agarose ((Invitrogen, Life technology, Grand Island, NY, USA), 50 µg/mL chloramphenicol, 25 µg/mL kanamycin, and 0.1 mM IPTG) and then incubated for 3 days at 37°C. The plates were stained with Lugol's iodine solution at room temperature.

### 1.4. Sequence Analysis and Phylogenetic Tree Construction

The amino acid sequences of four GH16 family β-agarases were obtained via a BLAST search of the NCBI database (http://www.ncbi.nlm.nih.gov). The amino acid sequences of individual open reading frames were aligned using Clustal X, and conserved residues were highlighted using the Clustal X color scheme. Using UPGMA, an unrooted phylogenetic tree of GH16 family members was constructed based on amino acid sequence similarities (Sneath and Sokal, 1973).

### 1.5. Cell Lysate Preparation, Protein Quantitation, Sodium Dodecyl Sulfate-Polyacrylamide Gel Electrophoresis (SDS-PAGE), and Western blot analysis

To identify and localize the recombinant enzyme proteins produced by the transformants, cells were suspended in 40 mM Tris-HCl buffer (pH 8.0), sonicated using 50 short bursts of 2 seconds each, extracted at 4°C for 30 min, and fractionated into three fractions (total, soluble, and insoluble cell fractions), as previously described. Quantification of protein concentrations of cell lysate was performed using a Micro BCA kit (Pierce, Rockford, IL, USA). Equal amounts of cell lysates (15 µg) were electrophoresed on 8% SDS-polyacrylamide gels according to the method of Laemmli. The gels were stained with Coomassie Brilliant Blue (CBB) R-250 to detect protein bands. For Western blot analysis, equivalent amounts of *E. coli* cell lysates and culture supernatants were subjected to SDS-PAGE and then electro-transferred onto Immobilon-P membranes as described previously. Protein detection was performed using the ECL Prime Western blotting kit according to the manufacturer's instructions.

### 1.6. Purification of GH16B β-Agarase

To recover recombinant His-tagged GH16B β-agarase from *E. coli* transformant cultures (300 mL), the supernatant was obtained after centrifugation (8000 rpm, 20 min). The culture supernatant was mixed with ammonium sulfate (30% saturation), and the mixture was stirred overnight at 4°C to precipitate GH16B β-agarase. The precipitate was collected by centrifugation (20,000 rpm, 20 min), dissolved in 10 ml of 10 mM Tris-HCl buffer (pH 7.5), and dialyzed against 10 mM Tris-HCl buffer (pH 7.5) at 4°C for 3 hours. To purify GH16B β-agarase, the dialyzed recombinant His-tagged GH16B β-agarase solution was subjected to immobilized metal affinity chromatography (IMAC) using Ni-NTA resin as described previously.

### 1.7. Agarase Activity Assay

Quantitative analysis of GH16B β-agarase activity was performed using the DNS method, which detects reducing sugars released from agarose. The enzyme solution (100 µL) was mixed with an equal volume of 0.8% melted agarose in an appropriate buffer. The content of reducing sugars formed in the reaction mixture was determined colorimetrically using DNS reagent after 30 min of incubation at 50°C. The amount of enzyme that produced reducing power equivalent to 1 µmol of D-galactose per min was defined as one unit of enzymatic activity.

The optimal temperature was determined using 50 mM Tris-HCl buffer (pH 7.0) for 30 min over a temperature range of 25 to 70°C. The optimal pH was determined using 50 mM Mcllvaine buffer (pH 40 to 7.5) and 50 mM Tris-HCl buffer (pH 7.5 to 10.0). For temperature and pH stability assays, aliquots of enzyme were preincubated at the indicated temperature and pH for the indicated time, and then the relative enzyme activity was measured under standard assay conditions (30 min, 50°C, 50 mM Tris-HCl buffer, pH 7.0). The kinetic parameters of GH16B β-agarase were determined by adding a known amount of the enzyme solution to the agarose substrate solution (1 to 5 mg/mL agarose in 50 mM Tris-HCl, pH 7.0). The Lineweaver-Burk equation, generated via GraphPad Prism 8 statistical package (GraphPad Software Inc., USA), was used to calculate Km and *Vmax* values. To examine the effects of metal ions, reducing agent, and chelator EDTA on enzymatic activity, various metal ions (CaCl₂, CuSO₄, KCl, MgCl₂, MnCl₂, MnSO₄, and NaCl), TCEP, or EDTA were used at the indicated concentrations under standard analytical conditions.

### 1.8. Production of Antibody against GH16B β-Agarase

To prepare mouse polyclonal antibody specific for GH16B β-agarase, purified recombinant GH16B β-agarase solution (120 µg/300 µL PBS) was mixed with an equal volume of complete Freund's adjuvant, and the mixture (20 µg/100 µL) was injected intraperitoneally into mice. For secondary, tertiary, and quaternary immunization, a purified GH16B β-agarase solution was mixed with an equal volume of incomplete Freund's adjuvant and injected intraperitoneally into the mice every 10 day. Five days after quaternary immunization, the mice were bled. C57BL/6J male mice (6-weeks-old) were purchased from the JA Bio (Suwon-si, Gyeonggi-do, Korea) and maintained at the Animal Resources Center in Kyungpook National University (Daegu, Korea) under specific pathogen-free conditions. The experiment was performed in accordance with the guidelines for the management and use of experimental animals after obtaining approval from the Animal Experimental Ethics Committee of Kyungpook National University (KNU2020-0025).

### 1.9. Thin Layer Chromatography (TLC)

TLC analysis was performed for GH16B β-agarase-catalyzed hydrolysates of agarose, NAOS, or AOS on Silica Gel 60 aluminum plates, which were developed with n-butanol-ethanol-H₂O [3:2:2 (v/v)]. To detect oligosaccharides, a visualization solution (containing 0.2% (w/v) naphthoresorcinol, 10% (v/v) H₂SO₄, ethanol) was sprayed, followed by heating at 80°C. NA2 and the NAOS standard mixture containing NA2-NA18 were used as standards.

### 1.10. Analysis of enzymatic reaction products using matrix-assisted laser desorption/ionization time-of-flight/time-of-flight mass spectrometry (MALDI-TOF/TOF MS)

MALDI-TOF/TOF MS was performed according to previously described methods to analyze the hydrolytic products of agarose or AOS through enzymatic reactions. The matrix-assisted laser desorption/ionization tandem time-of-flight mass spectrometry (MALDI-TOF/TOF MS) was performed in the positive ion reflectron mode using the ultrafleXtreme system (Bruker Daltonics). The purified reaction product was dissolved in water, 1 µL of the dissolved reaction product was spotted onto a stainless steel target plate, and 0.3 µL of 0.01 M NaCl and 0.5 µL of 50 mg/mL 2,5-dihydroxybenzoic acid in 50% acetonitrile were added thereto. The spots were rapidly dried under vacuum for homogeneous crystallization. Each acquired spectrum represents the combined signal from 800 laser shots at each of 3 random locations on the spot, totaling 2,400 laser shots, using a 1-kHz laser. The laser attenuator offset and range were set at 68% and 15%, respectively, with the laser focus set at 50%. Mass spectra were recorded over an m/z range of 0 to 5,100. To obtain high-resolution data, the detector sampling rate was set at a maximum of 4.00 giga samples/s, and the detector gain was set at 4.0×. Mass spectra were externally calibrated using maltooligosaccharides isolated from commercial beer. A ladder of hexose polymers, spaced 1 hexose unit (162.053 Da) apart, provided comprehensive coverage of the entire mass acquisition range, enabling the accurate mass calibration of the MALDI-TOF/TOF MS just prior to the sample analysis. Raw MS data were processed with FlexAnalysis software (version 3.3; Bruker Daltonics). MS peaks were filtered with a signal-to-noise ratio of 3.0 and manually inspected to detect the formation of sodium, potassium, or other common adducts. All peaks were then deconvoluted, and a list of all neutral masses in the samples was generated, with the abundances represented by mass spectral peak intensities.

### 1.11. Purification of NA4 and NA6 by Size-Exclusion Column Chromatography

To purify NA4 and NA6 from the hydrolytic products of agarose, 9% melted agarose (20 mL) was treated with GH16B β-agarase under optimal conditions (1 mM MnCl₂ and 10 mM TCEP, 50°C, 50 mM Tris-HCl, pH 7.5) for 14 hours, followed by freeze-drying. The freeze-dried samples were dissolved in deionized water and subjected to size-exclusion chromatography using a Sephadex G-15 column (I.D. 1.35 x 120 cm) equilibrated with distilled water. 2 mL of each fraction eluting in deionized water were collected. TLC was used to identify fractions containing NA4 and NA6, which were then collected and lyophilized to dryness.

### 1.12. Statistical Analyses

Unless otherwise specified, the data represent at least three independent experiments. All data are expressed as the mean±standard deviation (SD, n≤3 per group). Statistical analyses were performed using Student's t-test to compare the data between two groups, whereas one-way analysis of variance followed by Dunnett's multiple comparison test was used to compare three or more groups. P values of <0.05 were considered to indicate statistical significance. Statistical analysis was conducted using SPSS Statistics version 23 (IBM, Armonk, NY, USA).

### 2. Results

### 2.1. Production of four Cellvibrio sp. KY-GH-1-derived GH16 family β-agarases as C-terminal His-tagged recombinant proteins using E. coli expression system and pET-30a vector

A previous study on whole genome sequence analysis of an agarolytic bacterium, *Cellvibrio* sp. KY-GH-1, revealed the presence of four GH16 β-agarase genes (GH16A, GH16B, GH16C, and GH16D) (Kwon et al., 2019). As shown in FIG. 1, GH16A has an open reading frame (ORF) encoding 477 amino acids (aa) that constitute a 52.0-kDa protein (GH16A β-agarase), and GH16B has an ORF encoding 597 aa that constitute a 63.8-kDa protein (GH16B β-agarase). In addition, GH16C has an ORF encoding 590 aa that constitute a 64.3 kDa protein (GH16C β-agarase). In contrast, GH16D has an ORF encoding 998 aa that constitute a 108.1 kDa protein (GH16D β-agarase), indicating that the molecular weight of GH16D β-agarase is much larger than those of the other three isoenzymes. The ORF amino acid sequence of GH16B β-agarase showed 51.7%, 52.0%, and 41.0% identity with GH16A, GH16C, and GH16D β-agarases, respectively. This indicates low homology among the four GH16 family β-agarases in the *Cellvibrio* sp. KY-GH-1 strain.

Based on these data, recombinant His-tagged GH16 β-agarases expressed in an *E. coli* expression system were used to examine which of the four GH16 family β-agarases has the most prominent endolytic hydrolytic activity for the agarose substrate. *E. coli* transformants expressing individual GH16A, GH16B, GH16C, and GH16D β-agarases were cultured on LB agarose solid medium plates containing 0.5 mM IPTG for 2 days, and then stained with Lugol's iodine solution. Agarose-degrading activity, evidenced by a bright clear zone around the colonies, was highest in the transformant expressing GH16B β-agarase (FIG. 2A). 0.5 mM IPTG was added to the culture media of individual *E. coli* transformants expressing GH16A, GH16B, GH16C, and GH16D β-agarases to induce protein expression. To examine whether the recombinant enzymes expressed intracellularly were secreted into the extracellular culture media, the cell cultures were centrifuged to prepare the culture supernatants as extracellular fractions. The collected cell pellets were processed to obtain total, soluble, and insoluble intracellular fractions, respectively. Equal amounts of each fraction were taken and subjected to 8% SDS-PAGE to examine the intracellular solubility/insolubility ratio and extracellular secretion rate of each recombinant enzyme. As shown in FIG. 2B, GH16A β-agarase, GH16C β-agarase, and GH16D β-agarase were predominantly detected in the insoluble inclusion bodies. On the other hand, GH16B β-agarase was predominantly detected in culture supernatants, indicating that the recombinant GH16B β-agarase expressed in the *E. coli* transformant were secreted extracellularly.

These results demonstrated that, among the four GH16 β-agarases (GH16A, GH16B, GH16C, and GH16D) which are the components of the agarose-degrading enzyme machinery of the *Cell vibrio* sp. KY-GH-1 strain, GH16B β-agarase is an endolytic β-agarase that plays a key role in agarose liquefaction as a component of the agarose-degrading enzyme machinery. Furthermore, these results suggest that GH16B β-agarase in the *Cell vibrio* sp. KY-GH-1 strain essentially functions as an extracellular enzyme.

### 2.2. Comparison of amino acid sequences of GH16B β-agarases with amino acid sequences of related enzymes from other sources

The amino acid sequence of GH16B β-agarase from *Cell vibrio* sp. KY-GH-1 was aligned with other GH16 family β-agarase sequences using the Clustal X program. As shown in FIG. 3A, analysis of the amino acid sequence of KY-GH-1 GH16B β-agarase 597 aa could confirm the presence of an N-terminal signal peptide sequence (22 aa). *Cellvibrio* sp. pealriver (GenBank accession No. WP_04962945.1), *Cell vibrio* sp. OA-2007 (Genbank accession No. WP_062064993.1), *Cell vibrio* sp. BR (Genbank accession No. WP_007640762.1) and *Cellvibrio* sp. BR (GenBank accession No. WP_007640762.1) also appeared to commonly contain the signal peptide sequence at their N-terminal region, supporting the possibility that these GH16 family β-agarases are produced in agarolytic *Cellvibrio* species as extracellular enzymes to degrade agarose. Meanwhile, the overall amino acid sequence of *Cellvibrio* sp. KY-GH-1 GH16B β-agarase (GenBank accession No. QEY14910.1) showed 100% identity with that of *Cellvibrio* sp. KY-YJ-3 GH16B β-agarase (GenBank accession No. QEY12839.1). The amino acid sequence of KY-GH-1 GH16B β-agarase also shared 98.8%, 96.0%, 95.0%, and 79.6% similarity with that of non-marine agarolytic bacteria, such as *Cellvibrio* sp. pealriver, *Cellvibrio* sp. OA-2007, *Cellvibrio* sp. BR, and uncultured bacterium (GenBank accession No. AAP49316.1), respectively, indicating a high degree of homology among *Cellvibrio* GH16B β-agarases. The amino acid sequence of GH16B β-agarase shared 53.1%, 52.1%, 50.9%, and 50.8% similarity with that of marine agarolytic bacteria, including *Pseudomonas* sp. ND137 (GenBank accession No. BAD88713.1) [Aoki and Kamei, 2006], *Marinimicrobium aglyticum* (GenBank accession No. WP_036188483.1), *Gilvimarinus chinensis* (GenBank accession No. WP_0208740.1), and *Simiduia agarivorans* (GenBank accession No. WP_015048661.1), respectively.

The rooted phylogenetic relationships of individual GH16B β-agarases revealed that the GH16B β-agarase of *Cellvibrio* sp. KY-GH-1/KY-YJ-3 is more closely associated with that of *Cellvibrio* sp. pealriver, *Cellvibrio* sp. OA-2007, *Cellvibrio* sp. BR, and uncultured bacterium isolated from nonmarine sources than with that of *Pseudomonas* sp. ND137, *M. agarilyticum, G. chinensis,* and S. *agarivorans* isolated from marine sources (FIG. 3B). Meanwhile, alignment of the GH16B β-agarase sequence with its sequence similarity revealed that GH16B β-agarase homologs exist in all agar-degrading bacteria, suggesting that GH16B β-agarase that endolytically degrades the β-1,4-linkages of agarose in agar-degrading bacteria is an essential component of the agar-degrading enzymatic machinery.

### 2.3. Enzymatic characteristics of GH16B β-agarase

Because the amino acid sequence analysis data revealed the presence of a putative N-terminal 22-aa signal sequence in GH16B β-agarase, the present inventors investigated whether the signal sequence is important for recombinant GH16B β-agarase secretion and/or solubility in the *E*. *coli* expression system. In this regard, protein expression was induced by adding 0.5 mM IPTG to the culture of each *E*. *coli* transformant expressing GH16B β-agarases with or without the 22-aa signal peptide sequence, and the amounts of the two GH16B β-agarases distributed in the intracellular and extracellular fractions of the transformant were compared by Western blot analysis using anti-GH16B β-agarase and anti-His-tag antibodies.

As shown in FIGS. 4A and 4B, GH16B β-agarase with the 22-aa signal peptide sequence was prominently detected in the extracellular culture supernatant in the soluble form; however, GH16B β-agarase without the signal peptide sequence was not secreted and remained in the intracellular portion, where it was exclusively detected in the insoluble form. The intracellular and extracellular distribution patterns of GH16B β-agarase in the transformant, detected by Western analysis using anti-GH16B β-agarase, were consistent with those detected by Western analysis using anti-His-tag antibodies. Under the same conditions, β-galactosidase, a well-known intracellular enzyme in *E. coli,* was also detected in the extracellular culture supernatant of the transformant expressing GH16B β-agarase with the 22-aa signal peptide sequence, but not in that of the transformants expressing GH16B β-agarase without the 22-aa signal peptide sequence. These results suggest that expression of GH16B β-agarase with the 22-aa signal peptide sequence can induce extracellular secretion of GH16B β-agarase itself as well as extracellular leakage of some intracellular proteins, including β-galactosidase. As a result of purifying the His-tagged GH16B β-agarase from the culture supernatant using ammonium sulfate precipitation (30% saturation), it was shown that most of the proteins, except for three proteins, including GH16B β-agarase (63.8 kDa) and unknown proteins (about 37.5 kDa and about120 kDa), were excluded by the ammonium sulfate precipitation procedure. Subsequent Ni-NTA purification resulted in the detection of a single band on 8% SDS-PAGE, indicating the isolation of the recombinant protein with high purity (FIGS. 4C and 4D).

To determine the utility of recombinant GH16B β-agarase for the production of NA4 and NA6 in large amounts from agarose, the enzymatic properties thereof were investigated. When the activity of GH16B β-agarase was measured at various temperatures, the highest activity was detected at 50°C, and more than 80% of the maximum activity was retained over the range of 25 to 60°C (FIG. 5A). These results indicate that the optimal temperature for the enzymatic activity of GH16B β-agarase is slightly higher than the temperature (20°C) at which the *E. coli* transformants are cultured to produce recombinant GH16B β-agarase as an extracellular soluble protein secreted into the culture supernatant. The enzymatic activity was observed over a wide pH range of 5.0 to 8.0 (optimum pH 7.0) (FIG. 5B). The enzyme was stable up to 50°C and retained about 90% and about 80% of its maximum activity for 3 h and 14 h, respectively; however, most of the enzymatic activity was lost after treatment at 55°C for 14 h or 60°C for 3 h, indicating that GH16B β-agarase is a highly thermostable enzyme (FIG. 5C). Although GH16B β-agarase was stable over the pH range of 5.0 to 8.0, it rapidly became inactive at an acidic pH of 4.0 or less and retained about 35% activity after 4-hour treatment at pH 10.0 (FIG. 5D). These results suggest that GH16B β-agarase is more stable at alkaline pH than at acidic pH. The *Km, Vmax, kcat,* and *kcat*/*Km* values of GH16B β-agarase for agarose were 14.40 mg/mL, 542.0 U/mg, 576.3 s⁻¹, and 4.80 × 10⁶ s⁻¹ M⁻¹, respectively (FIG. 5E).

The GH16B β-agarase activity was enhanced by 1.5 to 1.6-fold in the presence of 0.5 to 2.5 mM MnCl₂ and MnSO₄, with maximum enhancement in the presence of 1 mM MnCl₂, indicating that the enzymatic activity of GH16B β-agarase depends on Mn²⁺ (Table 1: Effect of metal ions and other chemicals on GH16B β-agarase activity)

**[Table 1]**

| Metal ion/chemical (mM) | | Relative activity (%)* | Metal ion/chemical (mM) | | Relative activity (%)* |
|---|---|---|---|---|---|
| Control | | 100 (± 1.15) | CaCl₂ | 0.5 | 96 (± 1.80) |
| | | | | 1.0 | 110 (± 6.85) |
| | | | | 2.5 | 103 (± 0.90) |
| MnCl₂ | 0.5 | 125 (± 1.40) | KCl | 0.5 | 103 (± 1.20) |
| | 1.0 | 163 (± 2.60) | | 1.0 | 109 (± 6.41) |
| | 2.5 | 155 (± 3.00) | | 2.5 | 104 (± 3.45) |
| MnSO₄ | 0.5 | 146 (± 1.95) | CuSO₄ | 0.5 | 103 (± 0.25) |
| | 1.0 | 156 (± 0.70) | | 1.0 | 97 (± 1.45) |
| | 2.5 | 156 (± 1.90) | | 2.5 | 74 (± 1.45) |
| MgCl₂ | 0.5 | 94 (± 2.80) | TCEP | 0.5 | 100 (± 1.55) |
| | 1.0 | 107 (± 0.80) | | 1.0 | 109 (± 2.85) |
| | 2.5 | 100 (± 3.55) | | 2.5 | 117 (± 6,00) |
| NaCl | 0.5 | 100 (± 0.30) | EDTA | 0.5 | 95 (± 4.75) |
| | 1.0 | 109 (± 5.33) | | 1.0 | 95 (± 2.02) |
| | 2.5 | 101 (± 2.25) | | 2.5 | 91 (± 4.35) |

| | | | | | |
|---|---|---|---|---|---|
| *Values represent the agarase activity (%) expressed in mean ± SD (*n =* 3) relative to the untreated control | | | | | |

However, the presence of CaCl₂, MgCl₂, KCl, NaCl, and EDTA at concentrations ranging from 0.5 to 2.5 mM had no significant effect on the enzymatic activity, whereas the presence of 2.5 mM CuSO₄ reduced the enzymatic activity to 74% of that in the control. Furthermore, at a concentration of 5 to 15 mM, the antioxidant TCEP, which is currently the most widely used SH reducing agent, could enhance the enzymatic activity in a dose-dependent manner by up to 1.6-fold (FIG. 6). In particular, in the presence of both 1 mM MnCl₂ and 5 to 15 mM TCEP, the GH16B β-agarases activity was further enhanced, and in the presence of both 1 mM MnCl₂ and 5 mM, 10 mM, and 15 mM TCEP, the enzyme activity was enhanced by up to 1.8-fold, 2.2-fold, and 2.6-fold, respectively, indicating a synergistic effect of Mn²⁺ and TCEP on GH16B β-agarase activity. In addition, these results suggest that the interaction of GH16B β-agarase with manganese ions required for appropriate enzymatic activity may be strong enough to resist the chelating action of EDTA.

### 2.4. Substrate degradation pattern of GH16B β-agarase

According to the reported literature, GH16 β-agarases are endo-acting enzymes that hydrolyze agarose to produce NA4/NA6 as end products [Michel et al., 2006; Chi et al., 2012]. To determine the substrate degradation mode, the substrates (agarose, NAOS mixture of NA2 to NA18, and AOS) were treated with GH16B β-agarose at different time points, and then the hydrolytic products were analyzed by TLC. As a result, in the hydrolysis pattern of the agarose substrate depending on the treatment time of GH16B β-agarase, endo-type degradation of agarose yielded NAOS with various degrees of polymerization (DPs) in the early stage, and yielded NA4/NA6 as end products in the late stage of the reaction (60 min) (FIGS. 7A and 7B). However, NA2 was not detected under these conditions. Also, TLC analysis of the enzymatic hydrolysates of NAOS mixture (NA2-NA18) as substrates revealed that NAOS equal to or larger than NA12-NA18 were the preferred substrates in the enzymatic reaction for producing NA4/NA6 (FIG. 7B). TLC analysis also revealed that an AOS mixture having various DPs can be degraded by GH16B β-agarase to yield NA4/NA6 as the major product (FIG., 7C). Concurrently, this reaction may produce a similar amount of agaropentaose (A5) that was presumably generated from the non-reducing end of AOS during enzyme-catalyzed AOS digestion.

When the GH16B β-agarase-catalyzed hydrolysate was further analyzed using MALDI-TOF/TOF MS, the detected masses of NA4 ([M+Na]⁺: 653) and NA6 ([M+Na]⁺: 959) were consistent with those calculated from their molecular formulas, as previously described (FIG.8A). Furthermore, MALDI-TOF/TOF MS analysis of the enzyme-catalyzed hydrolysate of AOS revealed that considerable amounts of A5 ([M+Na]⁺: 815), NA4, and NA6 were produced as end products of the enzymatic reaction (FIG. 8B). These results confirm that GH16B β-agarase endolytically hydrolyzes the agarose substrate to produce NA4 and NA6, whereas hydrolysis of the AOS substrate produces not only NA4/NA6 but also A5 from AOS by an enzymatic reaction.

Overall, these results suggest that recombinant GH16B β-agarase derived from *Cellvibrio* sp. KY-GH-1 can be used to produce NA4 and NA6 in large amounts from agarose, NAOS, or AOS.

### 2.5. NA4 and NA6 yield upon treatment of agarose with GH16B β-agarase under optimum conditions

To further examine whether GH16B β-agarase is efficient in producing NA4/NA6 from agarose, the present inventors investigated the maximum concentration of agarose (melted by heat treatment) that could be efficiently hydrolyzed into NA4 and NA6 by treatment with GH16B β-agarase under continuous magnetic stirring at 50°C, which is a temperature higher than the agarose gelling temperature (about 40°C). Various concentrations of agarose (5.0%, 7.0%, and 9.0%) were treated with GH16B β-agarase (1.6 µg/mL) under optimal reaction conditions (1 mM MnCl₂ and 10 mM TCEP, 50 mM Tris-HCl, pH 7.5, 50°C) for 14 hours, and individual hydrolysis products were analyzed by TLC. The complete hydrolysis of agarose into NA4/NA6 by enzymatic treatment was achieved up to 9.0% agarose concentration (FIG. 9A). Following complete hydrolysis of 9% agarose solution (20 mL) with 1.6 µg/mL GH16B β-agarase for 14 hours under these optimal reaction conditions, the reaction product was freeze-dried. The dried sample was dissolved in DI water and fractionated by size-exclusion chromatography using a Sephadex G-15 column. As a result of TLC analysis, NA4 was detected in fractions 30 to 34, and NA6 was detected in fractions 35 to 39 (FIG. 9B). NA4 and NA6 powders were recovered from the freeze-dried fractions 30 to 33 and 35 to 38, respectively. When an enzymatic hydrolysate of 1.8 g of agarose (9%, 20 mL) was fractionated by size-exclusion chromatography using a Sephadex G-15 column, about 650 mg NA4 and about 900 mg NA6 was recovered, representing approximately 85.3% of the theoretical maximum yield.

### 3. Discussion

The present inventors demonstrated that recombinant GH16B β-agarase derived from the agarolytic bacterium *Cell vibrio* sp. KY-GH-1 is produced extracellularly in the *E. coli* expression system and can hydrolyze the β-1,4-linkage of agarose to yield NA4/NA6 as end products. When a 9% agarose solution that had been melted by heat treatment was treated with recombinant GH16B β-agarases at 50°C with magnetic stirring for 14 hours and NA4/NA6 were purified from the hydrolysate by Sephadex G-15 column chromatography, NA4 and NA6 were recovered at about 85.3% of the theoretical maximum yield.

Previously, the present inventors observed that the genome of *Cell vibrio* sp. KY-GH-1 contains four putative GH16 β-agarase genes (β-CvAga16A, β-CvAga16B, β-CvAga16C, and β-CvAga16D), which may encode GH16A (477 aa, 52.0 kDa), GH16B (597 aa, 63.8 kDa), GH16C (590 aa, 64.3 kDa), and GH16D (998 aa, 108.1 kDa), respectively, which hydrolyze agarose to produce NA4/NA6 [Kwon et al., 2019]. As a result of expressing these four enzymes in *E. coli* transformants as recombinant His-tagged proteins, it was shown that only GH16B β-agarase with an N-terminal 22-aa signal peptide sequence was secreted into the extracellular culture medium in a soluble form and exerted efficient endolytic hydrolyzing activity, hydrolyzing agarose into NA4/NA6. On the other hand, when GH16B β-agarase without the 22-aa signal peptide sequence was expressed in *E. coli* transformants, it was not secreted extracellularly and accumulated primarily in the intracellular insoluble inclusion body fraction. These results indicate that among the four putative GH16 family β-agarases, GH16B β-agarase is the key enzyme responsible for the endolytic hydrolysis of agarose by the KY-GH-1 strain, and also suggest that the N-terminal 22-aa signal peptide sequence of GH16B β-agarase from *Cellvibrio* sp. KY-GH-1 may contribute to the extracellular secretion of GH16B β-agarase in *E. coli* transformants. It is generally known that most proteins destined for transport across bacterial cytoplasmic membranes are synthesized with a signal sequence at their N-terminus, which is then removed by a signal peptidase during or immediately after the membrane translocation event (Dalbey et al., 2012; Freudl, 2018). However, further research is needed to elucidate whether the recombinant GH16B β-agarase secreted extracellularly from *E. coli* transformants still retains the 22-aa signal peptide sequence. In addition, SDS-PAGE and Western blot analyses revealed that the overexpression of GH16B β-agarase with the 22-aa signal peptide sequence in *E*. *coli* transformants may cause extracellular leakage of not only GH16B β-agarase, but also several intracellular proteins, including β-galactosidase. Further research is also needed to elucidate the mechanism underlying this phenomenon.

The Clustal X program revealed that GH16B β-agarase homologs exist in all agar-degrading marine and nonmarine bacteria, suggesting that GH16B β-agarase is an essential component of the bacterial agar-degrading enzymatic machinery. Furthermore, as the enzymatic properties of none of these nine GH16B β-agarase homologs were examined, the GH16B β-agarase-related data reported in that study may provide insights into GH16 family β-agarases with significant homology to GH16B β-agarase.

The Km and Vmax values (14.40 mg/mL and 542.0 U/mg) of KY-GH-1 GH16B β-agarase for agarase were comparable to those of GH16 β-agarase (3.78 mg/mL and 11,400 U/mg) from *Catenovulum agarivorans* YM01 (Cui et al., 2014), GH16 β-agarase (3.9 mg/mL and 909.1 U/mg) from *Agarivorans* sp. LQ48 (Long et al., 2010), GH16 β-agarase (28.33 mg/mL and 88.25 U/mg) from *Pseudoalteromonas hodoensis* H7 (Park et al, 2015), GH16 β-agarase (7.7 mg/mL and 18.3 U/mg) from *Saccharophagus degradans* 2-40^{T} ((Kim et al., 2017), GH16 β-agarase (4.8 mg/mL and 517 U/mg) from *Microbulbifer* sp. JAMB-A94 (Ohta et al., 2004), GH16 β-agarase (2.33 mg/mL and 101 U/mg) from *Vibrio* sp. strain PO-303 (Dong et al., 2007), and the like. Furthermore, GH16B β-agarase appeared to be more catalytic than other related enzymes in terms of *kcat* and *kcat*/*Km* values (576.3 s⁻¹, and 4.80×10⁶ s¹ M⁻¹, respectively) for the agarose substrate.

Unlike the marine-origin β-agarase activity, which was reported to generally rely on the presence of Mg²⁺ and Na⁺ (An et al., 2018; Han et al., 2016; Xie et al., 2013), GH16B β-agarase activity was not significantly affected by the presence of MgCl₂ or NaCl. Instead, GH16B β-agarase activity was enhanced in the presence of Mn²⁺ or the reducing agent TCEP in a dose-dependent manner. A synergistic effect of Mn²⁺ and TCEP on GH16B β-agarase activity was observed, and the enzyme activity could increase up to 2.6-fold in the presence of 1 mM MnCl₂ and 15 mM TCEP. The Mn²⁺/TCEP-dependent enhancement of GH16B β-agarase activity was comparable to the Mn²⁺/TCEP-dependent enhancement of *Cellvibrio* sp. KY-GH-1 GH50A β-agarase activity (Kwon et al., 2020) or GH117A α-NABH activity (Jang et al, 2021). These previous and current study results indicate that the presence of Mn²⁺ and TCEP increases the activities of all three enzymes, which are essential components of the agarose-degrading enzyme machinery involved in the degradation of a polysaccharide agarose substrate into the monosaccharides L-AHG and D-Gal in the KY-GH-1 strain, a freshwater-derived agarolytic bacterium.

GH16B β-agarase was identified as a highly thermostable enzyme in that it retained about 90% of its maximum activity after treatment at 50°C for 3 h and about 80% of their maximum activity after treatment at 50°C for 14 h. Previously, a recombinant GH16 β-agarase YM01-3, derived from the marine bacterium, *C. agarivorans* YM01, was reported to exhibit optimal activity at 60°C and retained about 80% enzymatic activity after treatment at 50°C for 1 h, suggesting that it exhibited higher thermal stability than other reported GH16 β-agarases (Cui et al., 2014). These current and previous study results suggest that although the optimum temperature for GH16B β-agarase (50°C) from the KY-GH-1 strain is slightly lower than that for GH16 β-agarase YM01-3 (60°C), the thermal stability of GH16B β-agarase 50°C is significantly higher than that of GH16 β-agarase YM01-3.

Since the gelling temperature of agarose melted by heat treatment is approximately 40°C, the enzymatic properties of GH16B β-agarase confirmed by the optimum temperature (50°C) and thermostability (stable at 50°C for 14 hours) of GH16B β-agarase suggest that GH16B β-agarase of the present invention may be effectively used in an enzymatic process of converting an agarose solution, melted by heat treatment, into NAOS with low degrees of polymerization (DPs) by liquefying the same at 50°C, which is higher than the gelling temperature of agarose.

Consistent with previous study results showing that GH16 family β-agarases endolytically hydrolyze agarose to produce NA4 and NA6 as end products (Michel et al., 2006; Cantarel et al., 2009; Chi et al., 2012), TLC analysis of 0.8 µg/mL GH16B β-agarase-catalyzed hydrolysates of either 0.4% agarose or 1.0% NAOS, obtained after incubation at 50°C for 1 h, showed NA4 and NA6 as end products. On the contrary, TLC analysis of 1.6 µg/mL GH16B β-agarase-catalyzed hydrolysates of 5%, 7%, or 9% melted agarose substrate, obtained after incubation at 50°C for 14 h under continuous magnetic stirring, reveled complete agarose hydrolysis into the end products NA4 and NA6, with the detection of trace amounts of NA2. This suggests that GH16B β-agarase at a concentration of 1.6 ug/mL can further hydrolyze NA6 into NA4 and NA2. In addition, it was confirmed that, when an AOS mixture with various DPs, which could be prepared via the mild acid treatment of agarose (Kwon et al., 2020), was used as a substrate and hydrolyzed by treatment with GH16B β-agarase, NA4 and NA6 as well as agaropentaose (A5) were produced as end products.

TLC analysis of hydrolysates obtained after treating the agarose solution (5%, 7%, or 9%), melted by heat treatment, with GH16B β-agarase at a concentration of 1.6 µg/mL under optimal conditions (1 mM MnCl₂, 10 mM TCEP, 50 mM Tris-HCl, pH 7.0, 50°C) for 14 h under continuous magnetic stirring, indicated that GH16B β-agarase completely liquefied and hydrolyzed up to 9.0% agarose into the end products NA4 and NA6. When NA4 and NA6 contained in the obtained agarose hydrolysates were purified by size-exclusion chromatography using a Sephadex G-15 column, about 650 mg NA4/about 900 mg NA6 were obtained from the hydrolysates of 9% agarose (20 mL), and the recovery yield thereof was about 85.3% of the theoretical maximum yield. These results indicate that, since the optimal temperature for GH16B β-agarase is 50°C, which is higher than the gelling temperature of agarose (about 40°C), and the thermal stability thereof is also very good at 50°C, the recombinant GH16B β-agarase is a very useful enzyme that may be industrially used for enzymatic liquefaction of agarose and consequently for production of NA4/NA6 in large amounts from a high-concentration agarose substrate through a one-step liquefaction process.

In summary, the current results show that the recombinant endolytic GH16B β-agarase derived from the agarolytic bacterium *Cellvibrio* sp. KY-GH-1 could efficiently hydrolyze and liquefy up to 9% agarose in an endo-acting manner to produce NA4 and NA6. The optimal temperature and pH of GH16B β-agarase for agarose hydrolysis were 40 to 50°C and 7.0, respectively. The enzyme was stable at a temperature of up to 50°C and a pH of 5.0 to 8.0, and unstable at a temperature higher than 50°C and a pH outside the pH range of 5.0 to 8.0. In the presence of 1 mM MnCl₂ and 15 mM TCEP, the enzymatic activity was enhanced by 2.6-fold. The *Km, Vmax, kcat,* and *kcat*/*Km* values for agarose were 14.40 mg/mL, 542.0 U/mg, 576.3 s⁻¹, and 4.80×10⁶ s⁻¹ M¹, respectively. The enzymatic properties such as optimum temperature, thermal stability, and endolytic agarose-degrading activity of GH16B β-agarase show that GH16B β-agarase is very useful for converting high-concentration agarose, melted by heat treatment, into NA4 and NA6 in a single liquefaction process at 50°C, which is higher than the gelling temperature (about 40°C), and therefore, it is useful for mass production of NA4 and NA6 through this process. In addition, they suggest that, when an agarose liquefaction process using GH16B β-agarase is combined with a potent exolytic GH50 family β-agarase, it may be developed into a process of mass-producing NA2 from agarose, and when it is further combined with α-NABH that efficiently degrades agarose into the monosaccharides L-AHG and D-Gal, it may further be developed into an enzymatic process of mass-producing L-AHG from agarose.

### [Accession Number]

Depository authority: Korean Collection for Type Cultures (KCTC), Korea Research Institute of Bioscience and Biotechnology

### Accession number: KCTC13629BP

Deposit date: August 27, 2018

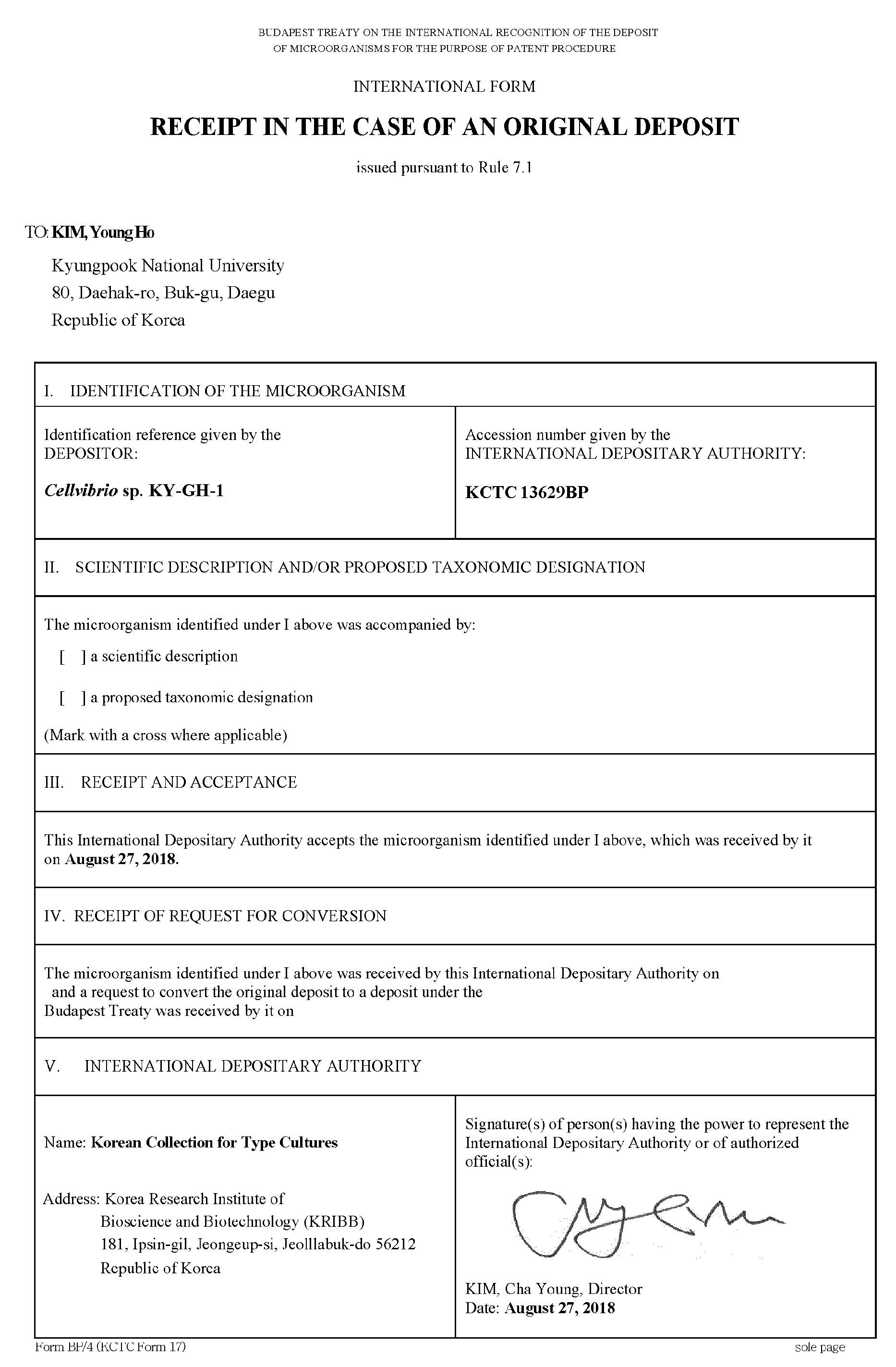
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KIM, Young Ho
Kyungpook National University
80, Daehak-ro, Buk-gu, Daegu
Republic of Korea

I. IDENTIFICATION OF THE MICROORGANISM
Identification reference given by the
DEPOSITOR:

Cellvibrio sp. KY-GH-1 | Accession number given by the
INTERNATIONAL DEPOSITARY AUTHORITY:

KCTC 13629BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 27, 2018.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Cha Young, Director
Date: August 27, 2018

Form BP/4 (KCTC Form 17) sole page

## Claims

1. A method for producing neoagarotetraose (NA4) and neoagarohexaose (NA6), **characterized by** enzymatically degrading a substrate by treating the same with a GH16B β-agarase comprising SEQ ID NO: 1.

2. The method of claim 1, wherein the GH16B β-agarase comprising SEQ ID NO: 1 is derived from a *Cellvibrio* sp. KY-GH-1 strain deposited under accession number KCTC 13629BP.

3. The method of claim 1, wherein the substrate is agarose or neoagarooligosaccharides (NAOS).

4. The method of claim 3, wherein a concentration of the agarose is 9 wt% or less.

5. The method of claim 1, wherein the treating is performed at a temperature ranging from 40 to 60°C.

6. The method of claim 1, wherein the treating is performed at a pH ranging from pH 5.0 to 8.0.

7. The method of claim 1, wherein the treating is performed by further adding Mn²⁺.

8. The method of claim 7, wherein the Mn²⁺ is MnCl₂ or MnSO₄.

9. The method of claim 7, wherein a concentration of the MnCl₂ or MnSO₄ is 0.5 to 2.5 mM.

10. The method of claim 1, wherein the treating is performed by further adding tris(2-carboxyethyl)phosphine (TCEP).

11. The method of claim 10, wherein s concentration of the tris(2-carboxyethyl)phosphine is 5 to 15 mM.
